# EUROPEAN PATENT SPECIFICATION

(11) **EP 0 644 422 B1**
(45) Date of publication and mention of the grant of the patent: **04.08.1999**
(21) Application number: 94113154.2
(22) Date of filing: 23.08.1994
(51) Int. Cl.: G01N 33/36, B65H 67/06, B65H 69/06, B65H 67/08

(54) **Package transport system**
Fadenwickeltransportsystem
Système de transport de paquets de fil

(30) Priority: 26.08.1993 JP 23574393; 10.11.1993 JP 30587993
(43) Date of publication of application: 22.03.1995
(73) Proprietor: MURATA KIKAI KABUSHIKI KAISHA, Kisshoin, Minami-ku Kyoto-shi 601 (JP)
(72) Inventor: Noda, Koshi, Joyo-shi, Kyoto-fu (JP); Tone, Shoichi, Sakyo-ku, Kyoto-shi (JP)
(74) Representative: Liedl, Christine, Dipl.-Chem.

(56) References cited:
- WO-A-91/04217
- CA-A- 851 412
- CA-A- 934 946
- DE-A- 2 831 233
- DE-A- 3 336 202
- DE-A- 4 041 715
- DE-A- 4 211 985
- DE-B- 2 831 242
- GB-A- 2 144 161
- US-A- 4 116 393
- PATENT ABSTRACTS OF JAPAN vol. 14, no. 46 (M-0926) 26 January 1990 & JP-A-01 275 376 (MURATA MACH LTD) 6 November 1989

## Description

The invention relates to a yarn package transport system acc. to the preamble of claim 1.

Automatically operating sampling stations for a yarn package transport system are known from US-A-4 116 393, CA-A-851 412, CA-A-934 946.

On the transport line, inspections for the appearance, cob-webbing, fluff and so forth are performed while packages are transported on a conveyor in a condition in which they are carried on peg trays. Where a sampling station for drawing out a yarn from each package and successively splicing the yarns is provided for the transport line and a test knitting machine is connected to the sampling station, sampling of a yarn of a predetermined amount is automatically performed and test knitting by the test knitting machine is performed subsequently to yarn splicing. Since a standard package and a color package are installed at the sampling station such that they can be changed over, a standard fabric for comparison or a color fabric for distinction can be automatically knit in intermediately of test knitting of packages.

In particular, when a yarn package is automatically doffed by an automatic doffer, each time a component for a doffing operation touches with a yarn, an end of the yarn is sometimes damaged. If such a damaged yarn end is sampled and inspected by test knitting or the like, the yarn cannot be evaluated accurately.

Further, on a drawing false twisting machine, oil is usually applied by an oiling apparatus to a yarn before it is rewound. However, since a yarn being wound is cut upon doffing with an automatic doffer and an end of the yarn is sucked in while doffing is performed, if the sucking force is weak, then there is the possibility that the yarn may be entangled with the oiling apparatus, and accordingly, cutting and sucking are performed after the yarn is removed from the oiling apparatus. Accordingly, since a first portion of a yarn on a surface layer is a yarn which has not been oiled, there is the possibility that the quality may not be evaluated accurately by test knitting or the like.

The problem underlying the invention is to provide a yarn package transport system by means of which error-free yarn sampling for quality inspections may be performed.

In accordance with the invention this object is accomplished by a surface layer yarn releasing station as defined in the characterizing part of claim 1.

The invention is based on the fact that irregular yarn which has been damaged upon doffing by an automatic doffer or to which oil is not applied is only present in the surface layer of packages.

The invention will now be described by way of examples and with reference to the accompanying drawings in which:

Figure 1 is an arrangement diagram of components of a package transport system of a first embodiment of the present invention.

Figure 2 is a front elevational view of essential part of the package transport system of the first embodiment of the present invention.

Figure 3 is a side elevational view of essential part of the package transport system of the first embodiment of the present invention.

Figure 4 is a view of a changing over procedure between synthetic yarn packages in the first embodiment.

Figure 5 is a view of a changing over procedure between synthetic yarn packages in the first embodiment.

Figure 6 is a view of a changing over procedure between a synthetic yarn package and a standard package or the like in the first embodiment.

Figure 7 is a view of a yarn splicing condition in the first embodiment.

Figure 8 is a developed view of a fabric knitted by test knitting in the first embodiment.

Figure 9 is a layout diagram of a testing factory in which the package transport system of the first embodiment is incorporated.

Figure 10 is a view showing the structure and operation of a yarn splicing apparatus in the first embodiment.

Figure 11 is a view showing essential part of a transport line in which a sampling apparatus of a second embodiment of the present invention is incorporated.

Figure 12 is a view showing the sampling apparatus for a yarn in the second embodiment.

Figure 13 is a sectional view of a rotation apparatus for a package in the second embodiment.

Figure 14 is a perspective view illustrating changing over of a yarn to a yarn splicing apparatus in the second embodiment.

Figure 15 is a perspective view of essential part of a synthetic yarn transport line in which the sampling apparatus is incorporated.

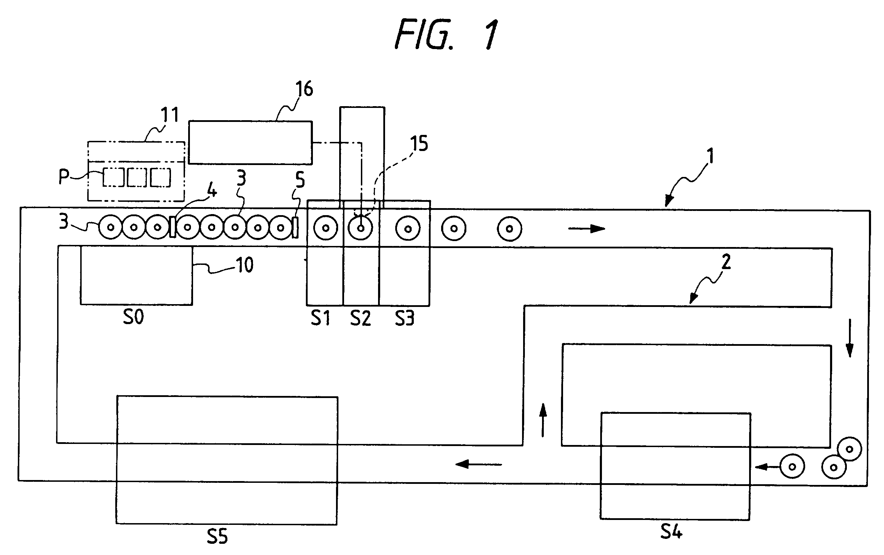

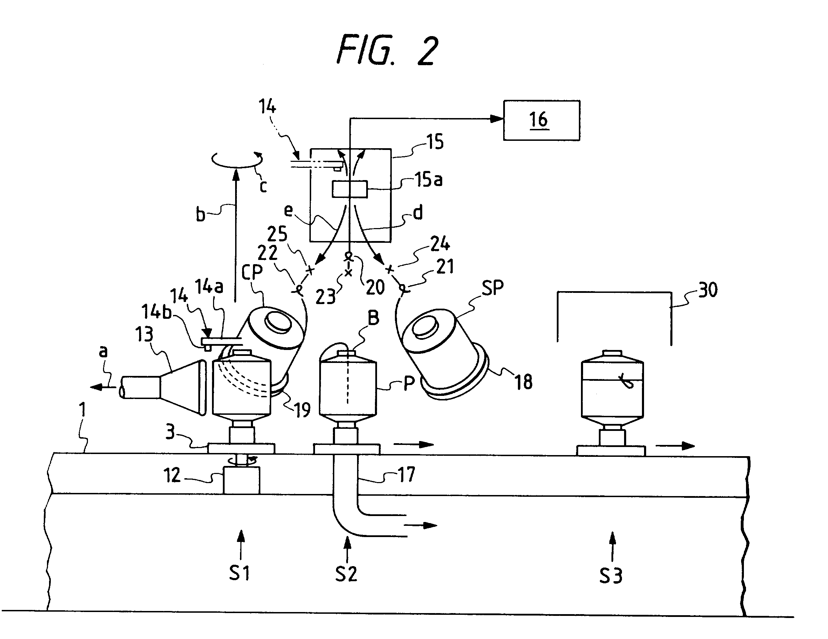

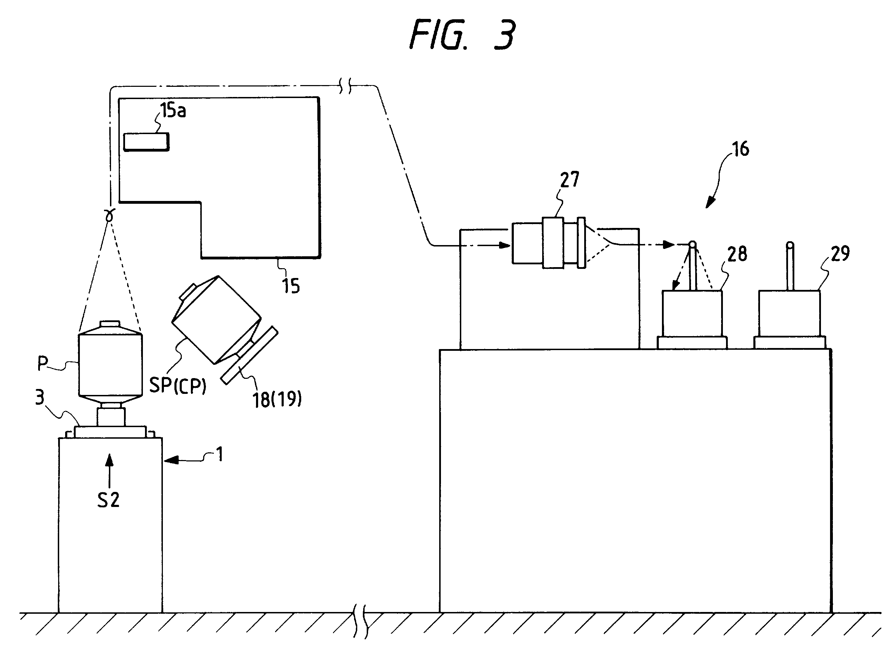

In the following, a first embodiment of the present invention will be described with reference to the drawings. Figure 1 is an arrangement diagram of components of a package transport system of the first embodiment; Figure 2 is a front elevational view of essential part of the package transport system; and Figure 3 is a side elevational view of essential part of the package transport system.

Referring to Figure 1, reference numeral 1 denotes a quadrangular transport line for circulation, and 2 a bypass line. The transport line 1 and the bypass line 2 are a connection of conveyors, on which peg trays 3 on which synthetic yarn packages can be carried are transported in the direction indicated by arrow marks. And, a peg tray 3 can be stopped at a predetermined position by means of a stopper 4 or 5 which can protrude above a conveyor.

A transfer station S0, a surface layer yarn releasing station S1, a sampling station S2, a surface layer yarn tying station S3, an inspection station S4, and a packing station S5 are arranged in order along the transport direction of the transport line 1. It is to be noted that the bypass line 2 extends from the downstream to the upstream of the inspection station S4 so that unacceptable synthetic yarn packages detected by the inspection station S4 can be temporarily stored on the bypass line 2 and synthetic yarn packages after retrieval can be returned to the transport line 1 again.

A transfer apparatus 10 is provided at the transfer station SO, and receives, at a peg shaft thereof with a chuck not shown, a synthetic yarn package P held on a carrier 11 of a ceiling transport vehicle, converts the posture of the synthetic yarn package P from a horizontal posture to a vertical posture, and transfers the synthetic yarn package P so as to position it onto the peg tray from above.

As shown in Figure 2, a rotation apparatus 12 for a peg tray 3, a suction mouth 13 and an upwardly and downwardly movable and pivotal sucker 14 are provided at the surface layer yarn releasing station S1. The suction mouth 13 has an elongate opening 13a opposed to a side face of a synthetic yarn package P to suck in the direction of an arrow mark a, and sucks an end of a yarn sticking to a surface layer of a synthetic yarn package which is rotated slowly by the rotation apparatus 12.

The sucker 14 includes a suction pipe 14b provided at an end of the pivotal lever 14a, and the pivotal lever 14a can be moved upwardly and downwardly as indicated by an arrow mark b by a slide apparatus not shown and can be pivoted in the direction indicated by an arrow mark c by drive means not shown. The sucker 14 at a solid line position has the suction pipe 14b positioned in the proximity of an upper end of the opening 13a of the suction mouth 13, and sucks, when a yarn sucked in and released by the suction mouth 13 while being traversed is traversed to an upper side of a synthetic yarn package P, the yarn in the suction mouth 13 and transfers it to the suction pipe 14b. Accordingly, the sucking force of the sucker 14 is set higher than the sucking force of the suction mouth 13. And, when the sucker 14 is lifted as indicated by the arrow mark b, the releasing direction of the yarn becomes the axial direction of the synthetic yarn package P, and the yarn is released rapidly irrespective of rotation of the synthetic yarn package P and a considerable amount of the yarn is sucked and carried to a dust box not shown. Further, when the sucker 14 is pivoted in the direction indicated by the arrow mark c so that it comes to an alternate two long and short dashes line position, the yarn can be introduced into a yarn guide 20 and a yarn splicing apparatus 15 which will be described below. The suction mouth 13 and sucker 14 constitute a yarn draw-out means, which draws out a yarn of a package P delivered on the transport line 1 and introduces the yarn to the yarn splicing apparatus 15.

The yarn splicing apparatus 15 is provided at the sampling station S2 of Figure 1, and a test knitting machine 16 is connected to the sampling station S2 so that a continuous yarn spliced by the yarn splicing apparatus 15 is introduced into the test knitting machine 16. As shown in Figure 2, a suction pipe 17 for the lower side of a peg tray 3, a receiving table 18 for a standard package SP on which a yarn of a standard quality is wound, a receiving table 19 for a color package CP on which a color yarn is wound, yarn guides 20, 21 and 22, clamp cutters 23, 24 and 25 and so forth are provided at the sampling station S2.

Further, as shown in Figure 3, a body 15a of the yarn splicing apparatus 15 is positioned on the interior side of a yarn path, and a pair of levers not shown positioned above and below the body 15a take, upon yarn splicing, yarn ends of two yarns into the body 15a by pivoting movements, twist the yarn ends by means of an air nozzle to make an entwinement of filaments to splice the yarns to each other and then cut excessive yarn ends by means of a cutter.

The test knitting machine 16 connected to the sampling station S2 includes a feeder 27, a first tubular knitting apparatus 28, and a second tubular knitting apparatus 29. The feeder 27 has a drum on which a yarn after spliced is to be wound, and draws in and stores a yarn of a predetermined amount and successively supplies the yarn to the tubular knitting apparatus 28 and 29. Each of the tubular knitting apparatus 28 and 29 knits a single yarn into a tubular shape, and since the size of stitches must be varied depending upon the thickness of the yarn, one of the first tubular knitting apparatus 24 for a thinner yarn and the second tubular knitting apparatus 25 for a thicker yarn is used.

Referring back to Figure 2, the clamp cutter 24 or 25 cuts a yarn from a standard package SP or a color package CP and holds the yarn on the package side, and further moves as indicated by arrow marks d and e to introduce the yarn into the yarn splicing apparatus 15. The clamp cutter 23 cuts the yarn extending from the synthetic yarn package P to the test knitting machine 16 and holds the yarn on the test knitting machine 16 side. And, the yarn on the synthetic yarn package P side is sucked from the top of the bobbin B by the suction of the suction pipe 17 so that the end of the yarn is suspended in the bobbin B.

The surface layer yarn tying station S3 includes a tying apparatus 30 which sucks and holds the end of the yarn in the bobbin and makes a knot 31 on an outer periphery of the package.

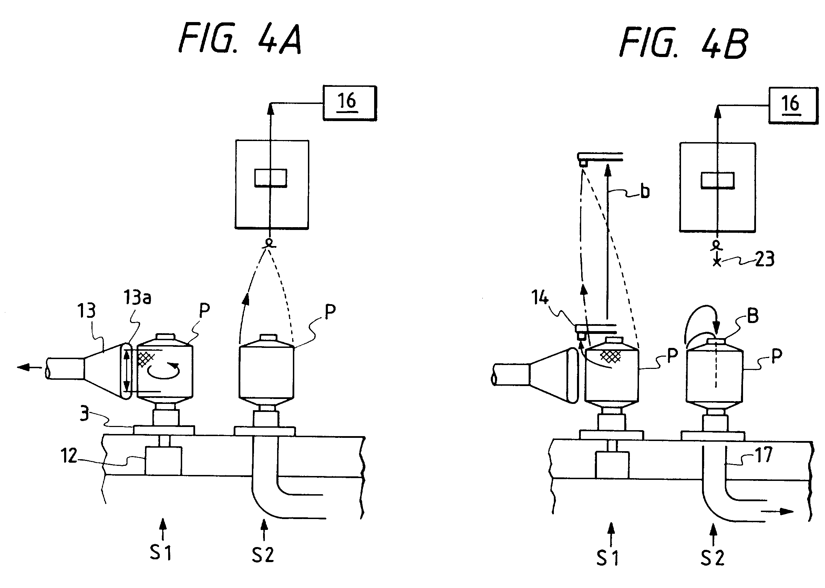

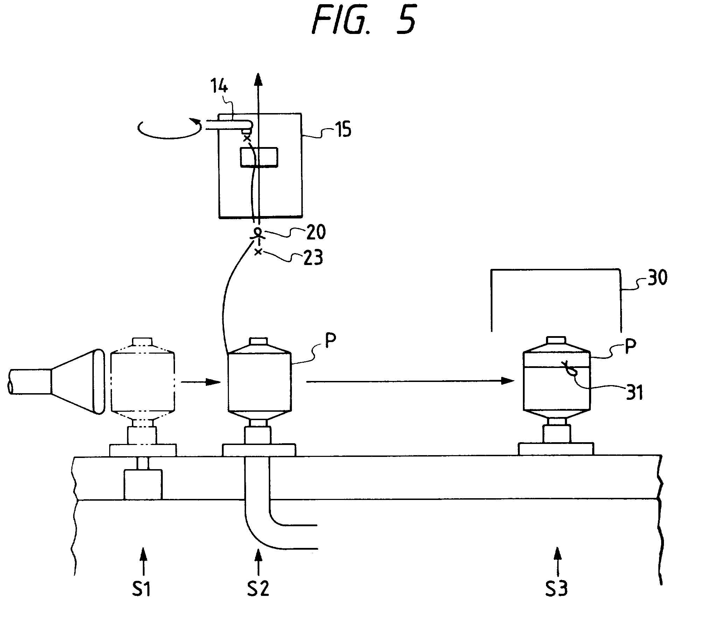

Subsequently, operation of essential part of the package transport system described above will be described with reference to Figures 4 to 6. Figures 4 and 5 illustrate a yarn splicing procedure between synthetic yarn packages P. In Figure 4A, a new synthetic yarn package P is supplied to the releasing station S1, and at the sampling station S2, a synthetic yarn package P is drawn out toward the test knitting machine 16. The synthetic yarn package P supplied to the releasing station S1 is rotated together with a peg tray 3 by the rotation apparatus 12, and an end of the yarn of a surface layer is sucked into the suction mouth 13. The yarn end thus sucked in is traversed by rotation of the package P.

In Figure 4B, the yarn end traversed by the suction mouth 13 is sucked in the sucker 14. And, when the sucker 14 is lifted as indicated by the arrow mark b, the yarn of the synthetic yarn package P is released rapidly, and a considerable amount of the yarn is abandoned into a dust box not shown by way of the sucker 14. The reason why a considerable amount of the yarn is abandoned is such as follows. Upon doffing by an automatic doffer, components for the doffing operation touch with the yarn so that an end of the yarn may possibly be damaged. If the damaged yarn end is used for test knitting, then the quality is not sometimes evaluated accurately. Further, in a draw texturing machine, oil is normally applied by an oiling apparatus to a yarn before it is wound. Further, upon doffing by an automatic doffer, a yarn being wound is cut and doffing is performed while an end of the yarn is sucked, and accordingly, if the force to such is weak, then the yarn may possibly be entangled with the oiling apparatus. Thus, the yarn is first removed from the oiling apparatus, and then cutting and sucking are performed. Accordingly, since the yarn end is a yarn not oiled, the quality may not possibly be evaluated accurately by test knitting. Therefore, sucking of the sucker 14 is performed until after a regular, oiled yarn free from a portion which may possibly be damaged upon doffing is obtained.

Meanwhile, the yarn of the synthetic yarn package P at the sampling station S2 of Figure 4B is cut by the clamp cutter 23, and the yarn extending to the test knitting machine 16 is held by the clamp cutter 23. The end of the yarn on the synthetic yarn package P side is drawn into the suction pipe 17 and sucked into the bobbin B so that it is put into a depending condition. Then, as shown in Figure 5, the synthetic yarn package P of the releasing station S1 is moved to the sampling station S2, and the yarn extending to the sucker 14 is introduced into the yarn guide 20 and the yarn splicing apparatus 15. And, yarn splicing to the yarn held by the clamp cutter 23 is performed so that the condition of Figure 4A in which a predetermined amount of the yarn is fed into the test knitting machine 16 is entered. Meanwhile, the synthetic yarn package P which has been at the sampling station S2 is moved to the surface layer yarn tying station S3, at which a tie knot 31 is formed by the yarn tying apparatus 30. By repetition of the operations described above, yarns necessary for test knitting are taken successively from a large number of synthetic yarn packages P into the test knitting machine 16.

By the way, when it is tried to evaluate the quality of a test knit, evaluation can be performed simply by contrast with a fabric knitted with a standard yarn.

While it is possible to conduct contrast with another fabric, contrast is easy where a standard is knitted intermediately in the test knit. Further, while a boundary of a test knit for each synthetic yarn package P can be distinguished by seeking a small projection at a splice of yarns, it is not easy to find out a splice from a continuous fabric. Thus, if a fabric of a color yarn is knit in, for example, for each 10 packages, it is easy to seek a splice within a limited range of 10 packages.

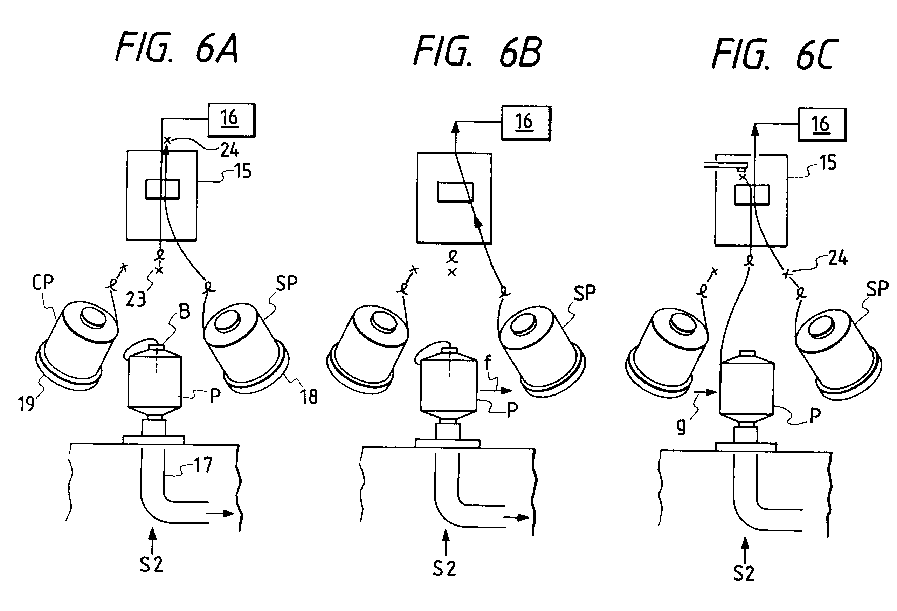

To this end, changing over between a standard package SP and a synthetic yarn package P, changing over between a color package CP and a synthetic yarn package P and changing over between a standard package SP and a color package CP in Figure 6A are performed at the sampling station S2.

Figure 6 illustrates an example of a changing over procedure from a synthetic yarn package P to a standard package SP. Referring to Figure 6A, the yarn of the synthetic yarn package P is cut by the clamp cutter 23, and the yarn extending to the test knitting machine 16 is held by the clamp cutter 23. An end of the yarn on the synthetic yarn package P side is sucked into a bobbin B by the suction pipe 17. And, the clamp cutter 23 holding the standard package SP is lifted as shown in Figure 6A to introduce the standard yarn into the yarn splicing apparatus 16. And, yarn splicing to the yarn extending to the test knitting machine 16 is performed.

And, a predetermined amount of the yarn from the standard package SP is taken into the test knitting machine 16 as shown in Figure 6B. It is to be noted that the synthetic yarn package P from which the yarn necessary for test knitting has been taken in is discharged as indicated by an arrow mark f. Subsequently, a new synthetic yarn package P is fed in as indicated by an arrow mark g, and an end of the yarn is introduced into the yarn splicing apparatus 15 in such a manner as described above. Simultaneously, the yarn of the standard package SP is cut and held by the clamp cutter 24. And, the standard yarn extending to the test knitting machine 16 and the yarn of the new synthetic yarn package P are spliced with each other, and taking in of the yarn from the synthetic yarn package P is performed.

It is to be noted that changing over between a synthetic yarn package P and a color package CP or changing over between a standard package SP and a color package CP is performed in a similar manner. Further, changing over of a color package CP can be performed also for each synthetic yarn package.

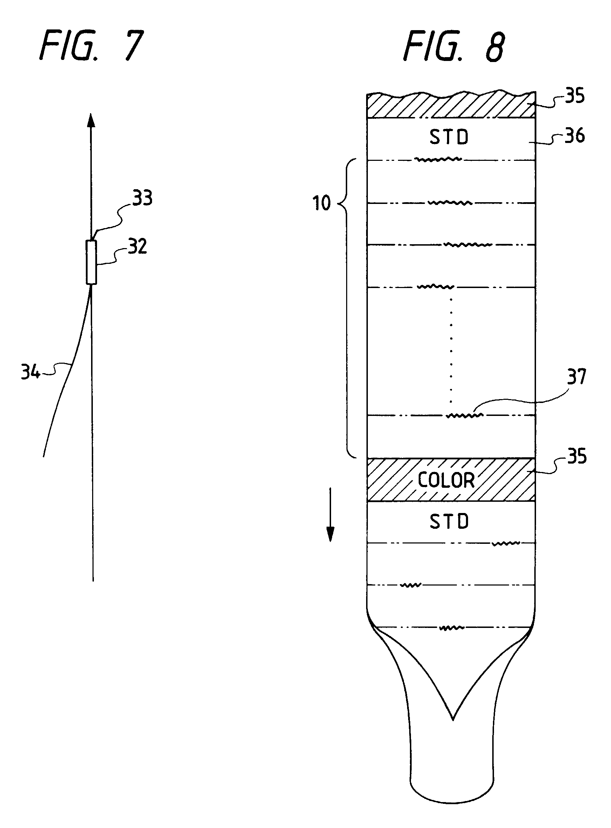

By the way, in tubular knitting, a boundary between synthetic yarn packages P or a boundary between a standard package SP and a synthetic yarn package P is discriminated from a splice between yarns. A method of making such splice clear will be described with reference to Figure 7. Referring to Figure 7, while a tail 33 of a splice 32 on the upstream in a feeding direction is cut as short as possible, another tail 34 on the downstream in the feeding direction depends long. If the yarn in this condition is knitted in, then a splice at a stitch appears not as a dot but as a line in a swollen condition and can be identified simply.

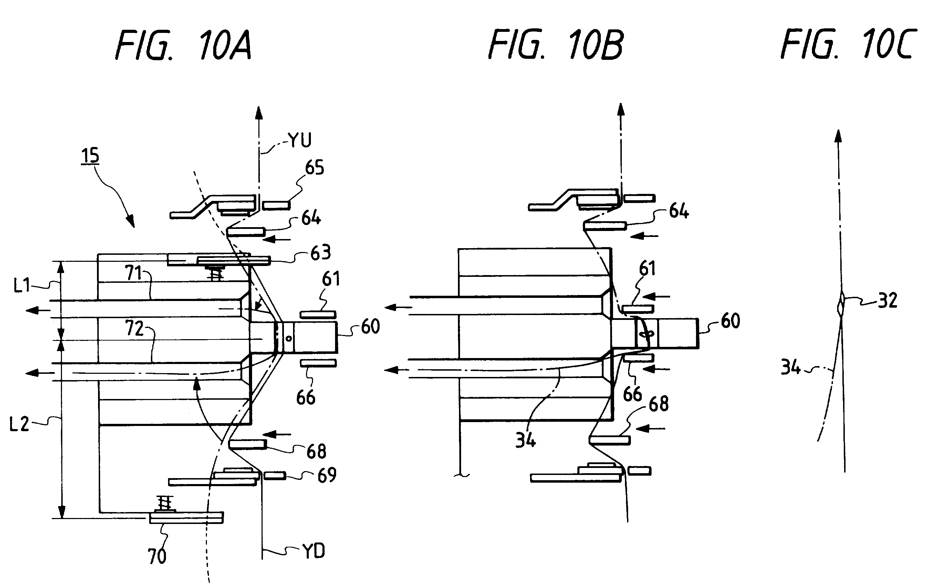

Subsequently, a yarn splicing apparatus for forming such spliced yarn portion 32 will be described with reference to Figure 10. Referring to Figure 10A, reference character YD denotes a lower yarn, and YU an upper yarn. And, the yarn splicing apparatus 15 includes a first upper yarn putting aside lever 61, a suction nozzle 71, a lower yarn cutter 63, a second upper yarn putting aside lever 64 and an upper yarn clamp 63 disposed in order on the upstream side of a yarn splicing member 60, and further includes a first lower yarn putting aside lever 66, a suction nozzle 72, a second lower yarn putting aside lever 68, a lower yarn clamp 69 and an upper yarn cutter 70 disposed in order on the downstream side of the yarn splicing member 60. Particularly, the positions of the upper and lower cutters 63 and 70 are different such that the distance L2 of the upper yarn cutter 70 to the yarn splicing member 60 is longer than the distance L1 of the lower yarn cutter 63 to the yarn splicing member 60. The ratio between L1 and L2 is equal to or higher than 1:2 and preferably set to approximately 1:3.

Subsequently, a yarn splicing method by the yarn splicing apparatus 15 will be described. Referring to Figure 10A, the upper yarn YU and the lower yarn YD are pushed in an arranged condition with tails thereof extended vertically into the yarn splicing member 60 by the second upper yarn putting aside lever 64 and the second lower yarn putting aside lever 68, and the upper yarn YU and the lower yarn YD are held by the upper clamp 65 and the lower clamp 69, respectively, whereafter the tails of the upper yarn YU and the lower yarn YD are cut by the lower yarn cutter 63 and the upper yarn cutter 70, respectively.

The suction nozzles 71 and 72 begin to operate before such cutting, and not only the longer tail 34 thus cut but also the shorter tail are sucked into the suction nozzles 71 and 72 so that they are put into a taut condition.

Referring to Figure 10B, the second upper yarn putting aside lever 64 and the second lower yarn putting aside lever 68 are further pushed in, and the tail of the lower yarn YD cut is almost drawn into the yarn splicing member 60 while the tail of the upper yarn YU cut is drawn into the yarn splicing member 60, but the tail 34 remains in a condition drawn by the suction nozzle 72 as shown in Figure 10B. Subsequently, the yarns are held down by the first upper yarn putting aside lever 61 and the first lower yarn putting aside lever 66 and then compressed air is jetted into the yarn splicing member 60 so that the tails are entangled with each other and are twisted with each other, thereby completing the yarn splicing.

Until after the yarn splicing is completed, the operations of the suction nozzles 71 and 72 continue, and it is prevented that the yarn splicing is influenced by twisting by the yarn splicing member 60 so that particularly the longer tail 34 becomes dispersed. And, if the operation of the suction nozzle 72 is stopped and the tail 34 is released from the condition of Figure 10B, then the downstream of the spliced yarn portion 32 remains as an independent tail 34 as shown in Figure 10C.

Referring to Figure 10B, the second upper yarn putting aside lever 64 and the second lower yarn putting aside lever 68 are further pushed in, and the tail of the lower yarn YD cut is almost drawn'into the yarn splicing member 60 while the tail of the upper yarn YU cut is drawn into the yarn splicing member 60. Subsequently, the yarns are held down by the first upper yarn putting aside lever 61 and the first lower yarn putting aside lever 66 and then compressed air is jetted into the yarn splicing member 60 so that the tails are entangled with each other and are twisted with each other, thereby completing the yarn splicing. And, if the tail 34 is released from the condition of Figure 10B, then the downstream of the spliced yarn portion 32 remains as an independent tail 34 as shown in Figure 10C.

Subsequently, an example of development of a fabric knitted by test knitting with a yarn having a spliced yarn portion whose tail on the downstream side is elongated will be described with reference to Figure 8. The test knitted fabric includes, in order to make a distinction further clear to facilitate counting of unacceptable locations, a color portion 35 knitted in with a color yarn each time one of yarns sampled from 10 synthetic yarn packages is knitted in. Further, also a standard portion 36 formed from a standard yarn is knitted in subsequently to the color portion 35 so that discrimination of the quality by comparison between a sampled portion and the standard portion 36 may be performed rapidly. Due to the presence of such color portions 35, at which position an unacceptable portion is present can be counted simply by eye measure. Further, since a splice of a yarn of a synthetic yarn package has a long tail, the spliced portion of a stitch becomes conspicuous as a line as at a splice 37. Accordingly, if unaccetable portions such as tight spot portions are present as a result of comparison with the standard portion 36, the number of such unacceptable portions is counted, and an unacceptable package can be specified. Further, such an ID as a bar code is applied to each peg tray for receiving a package from which a yarn is to be sampled, and the system is constructed such that, if the number of an unacceptable package is inputted, the unacceptable package is automatically identified by the inspection station S4 and discharged onto the bypass line 2.

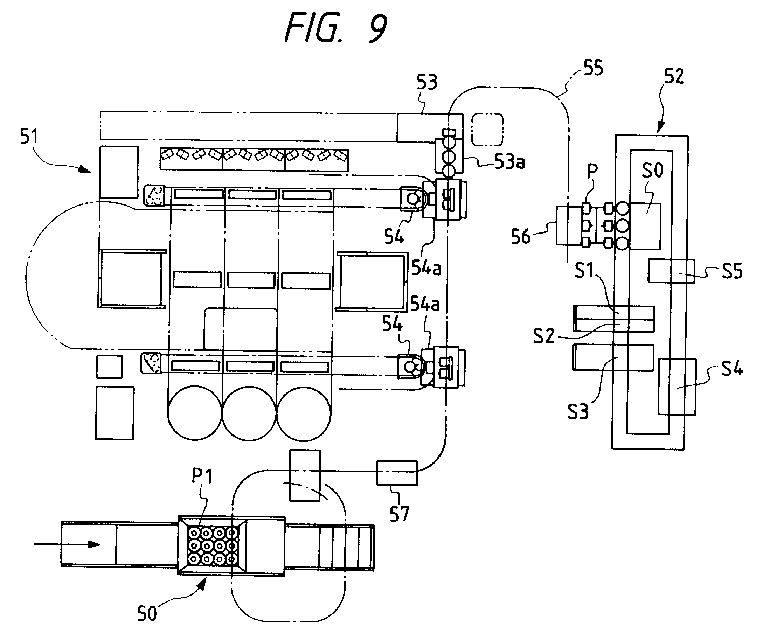

Further, an example of a layout of a drawing false twisting machine testing factory in which the synthetic fiber processing line described above is included will be described with reference to Figure 9. Reference numeral 50 denotes a supply yarn unpacking station, 51 a draw texturing machine, and 52 a synthetic yarn transport line. And, a supply yarn exchanging apparatus 53 and a synthetic yarn transport line 54 are disposed along the draw texturing machine 51. And, the supply yarn unpacking station 50, stations 54a at the opposite ends of the package carrier 54, a station 53a alongside a home position of the supply yarn exchanging device 53, and the transfer station S0 of the synthetic yarn processing line 52 are interconnected by way of a ceiling rail 55, and two ceiling transport vehicles 56 and 57 can travel along the ceiling rail 55 freely. The ceiling transport vehicle 56 transports synthetic yarn packages produced from the draw texturing machine 51 to the synthetic yarn transport line. The ceiling transport vehicle 57 supplies supply yarn packages P1 to the supply yarn exchanging apparatus 53 for the draw texturing machine 51. With the layout described above, also an inspection by test knitting is performed while synthetic yarn packages from the draw texturing machine are automatically inspected and packed on the synthetic yarn transport line.

It is to be noted that the inspection based on sampling described above is not limited to test knitting and an inspection of a crimp degree test by a text yarn tester or an inspection of a strength and elongation of a yarn or the like can be performed with a continuous yarn sampled from a large number of packages as described above. Accordingly, also the package of an object for inspection is not limited to a synthetic yarn package but may be a package of a spun yarn.

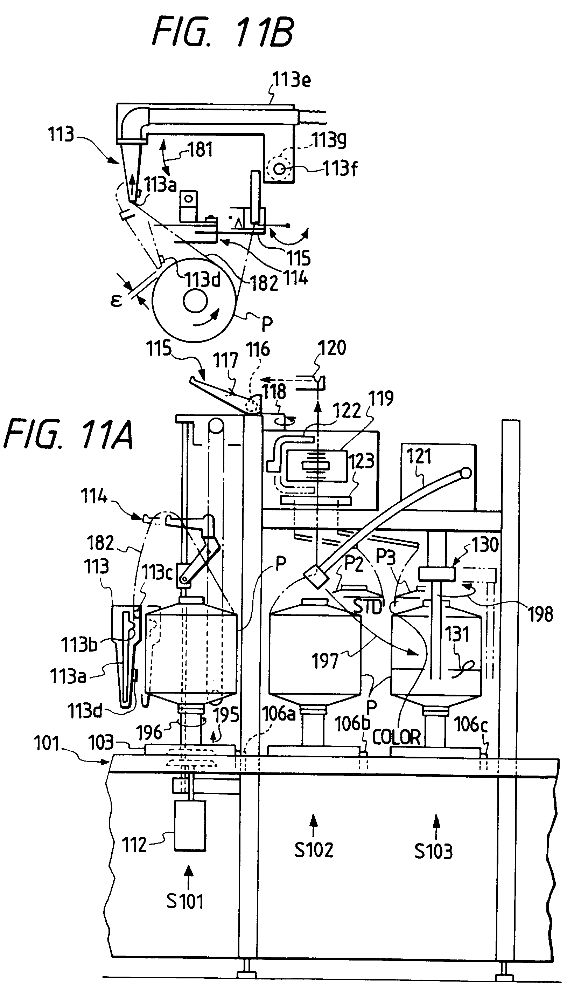

Subsequently, a second embodiment will be described with reference to the drawings. Figure 11 is a view showing a package transport line in which a sampling apparatus of the second embodiment is incorporated, and Figure 11A is a front elevational view and Figure 11B is a top plan view.

Referring to Figure 11A, a surface layer yarn releasing station S101, a sampling station S102 and a yarn tying station S103 are disposed in order intermediately of a transport line 101. Stoppers 106a, 106b and 106c are provided for protruding movement at the stations S101, S102 and S103 so that peg trays 103 on which packages P are fitted uprightly can be stopped there. The transport line 101 is constructed from slippable drive rollers provided in a row as shown in Figure 11A, and a chain conveyor is employed for the transport line 101 since a rotation apparatus 112 for lifting and rotating a package is provided at the surface layer yarn releasing station S101.

At the surface layer yarn releasing station S101, the rotation apparatus 112, a suction mouth 113 serving as first suction means, a movable first yarn catching lever 114 and a fixed second yarn catching lever 115 serving as lifting means, a suction pipe 116 serving as second suction means, a cutter 117, and a yarn putting aside lever 118 are provided. And, a yarn splicing apparatus 119 is provided at the sampling station S102. The components provided at the two stations S101 and S102 constitute a sampling apparatus. Meanwhile, a yarn spliced by the yarn splicing apparatus 119' is taken in over a predetermined length thereof by drawing in means on various inspection apparatus side.

The suction mouth 113 has an elongate opening 113a along a side face of a package P and is possible to suck, and sucks therein an end of a yarn sticking to a surface of a package P rotated by the tray rotation apparatus 112. While the surface layer yarn is wound by cross winding and the location at which an end of a yarn sticks is not fixed, the end of the yarn is sucked at some location of the elongate opening 113a. After the yarn end begins to be sucked, the yarn is sucked while the yarn end moves upwardly and downwardly in the opening 113a. And, the yarn entering in a tangential direction into the package P moves upwardly and downwardly rubbing the end of the opening 113a, and the yarn is arrested in a depression 113b located intermediately of the opening 113a. Further, a first sensor 113c for detecting a yarn in the depression 113b and a second sensor 113d for detecting that the opening 113a comes near to the package P are mounted. As shown in Figure 11B, the suction mouth 113 is provided fixedly on a pivotal lever 113e and can make pivotal motion as indicated by an arrow mark 181 around a shaft 113f by a motor 113g. And, the opening 113a can assume an alternate long and two short dashes line position (this position is detected by the second sensor 113d) at which it is nearer by a gap ε to the package P and a home position of a solid line. And, a yarn portion 182 extending from the package P to the opening 113a is caught and lifted by the first yarn catching lever 114 which will be described subsequently.

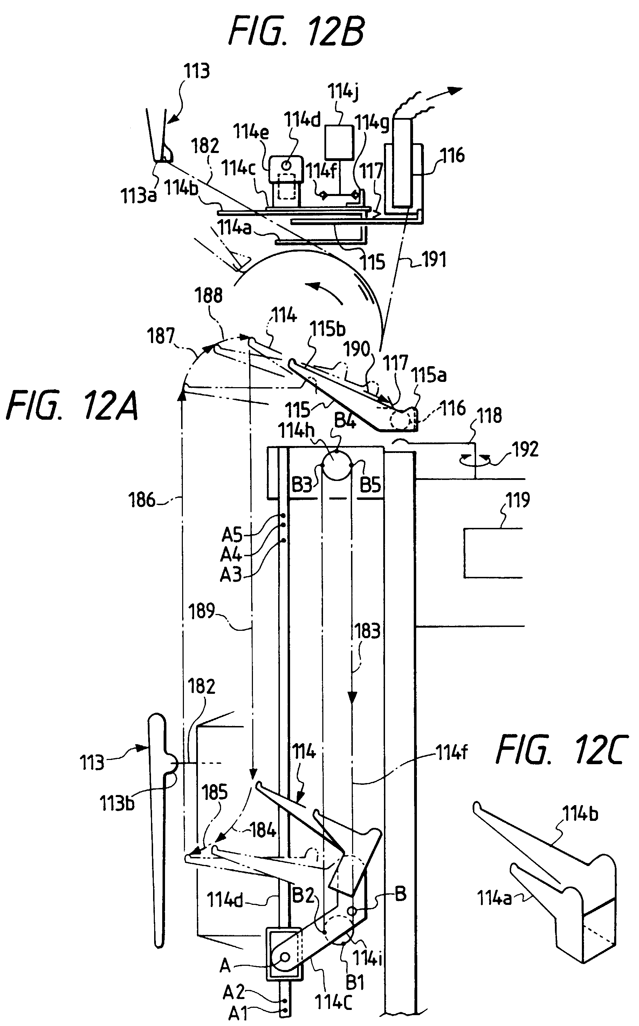

Subsequently, details of the first yarn catching lever 114 will be described with reference to Figure 12. Figure 12A is a front elevational view, Figure 12B is a top plan view, and Figure 12C is a perspective view of a body of the lever. As shown in Figure 12C, the lever body has a short lever 114a and a long lever 114b formed by bending into a channel shape as viewed in top plan, and is provided fixedly on a side face of an L-shaped connecting member 114c of Figure 12A. The connecting member 114c is supported at a lower end thereof for pivotal motion around a point A (refer to Figure 12A) on a sliding member 114e which slidably moves on a guide bar 114d, and the connecting member 114c is supported at an upper end thereof for pivotal motion around a point B (refer to Figure 12A) of a metal member connected to a chain 114f. As shown in Figure 12A, the chain 114f is wound around a driving sprocket wheel 114h and a driven sprocket wheel 114i and is rotated to travel by driving of a motor 114j (refer to Figure 12B).

The position of the first yarn catching lever 114 in a posture in which it is directed obliquely upwardly as indicated by a solid line is a home position. When the chain 114f is driven in the direction of an arrow mark 183 so that the point B comes to another position B1 along the driven sprocket wheel 114i, the point A comes to another point A1, whereupon the posture of the connecting member 114c is changed so that an end of the first yarn catching lever 114 moves as indicated by an arrow mark 184 to a gently inclined position. When the point B comes to a further position B2, the point A comes to a position A2 so that the end of the first yarn catching lever 114 moves as indicated by an arrow mark 185 to a horizontal posture. And, the first yarn catching lever 114 in the horizontal posture catches a yarn portion 182 (refer to Figure 12B) arrested at the depression 113b of the suction mouth 113 and raises the same as indicated by an arrow mark 186 until the point B comes to a position B3 and the point A comes to a position A3.

When the point B comes to a position B4 and the point A comes to a position A4, the end of the first yarn catching lever 114 is inclined so that the first yarn catching lever 114 rises as indicated by an arrow mark 187. When the point B comes to a position B5 and the point A comes to a position A5, the inclination of the first yarn catching lever 114 increases to its maximum as indicated by an arrow mark 188, and the yarn caught by the yarn catching lever 114 tends to slip down along the inclined face. And, the first yarn catching lever 114 in the inclined posture moves down while it remains in the posture. Intermediately of the movement of the point B of B3 → B4 → B5, a condition wherein the second yarn catching lever 115 is inserted between the short lever 114a and the long lever 114b of Figure 12C is reached, and the yarn portion rides over to the second yarn catching lever 115.

And, the yarn portion 182 (refer to Fig. 12B) slips down toward the depression 115a of the second yarn catching lever 115 as indicated by an arrow mark 190. Intermediately of the slipping down movement, the cutter 117 waits so that the yarn portion 182 is cut while simultaneously an end of the yarn is sucked into the suction pipe 116 and thus changes to a locus of such a yarn portion 191 as shown in Figure 12B. And, when the yarn putting aside lever 118 is pivoted by 180 degrees as indicated by an arrow mark 192, the yarn sucked in the suction pipe 116 is introduced into the yarn splicing apparatus 119. A yarn draw-out means which draws out a yarn of a package P delivered on the transport line 101 and introduces the yarn to the yarn splicing apparatus 119, comprises the suction mouth 113, the first yarn catching lever 114, the second yarn catching lever 115, the suction pipe 116, the cutter 117, the yarn putting aside lever 118, and the like.

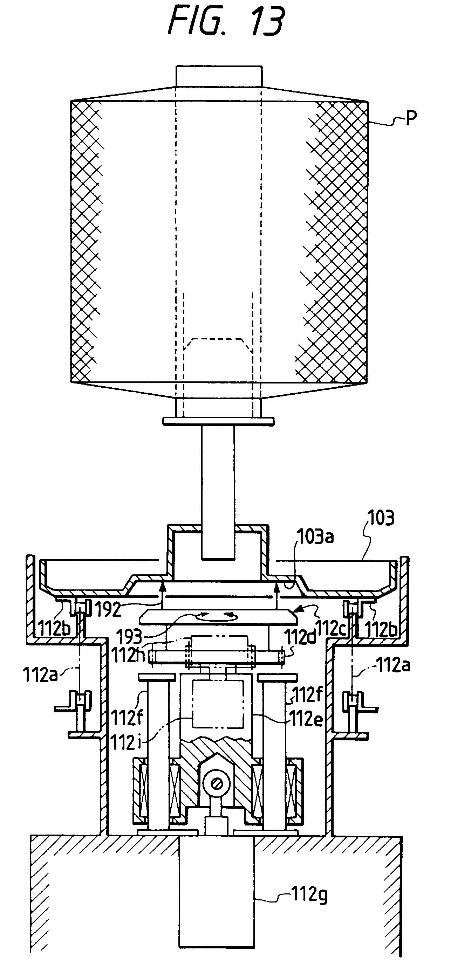

Subsequently, the structure of the rotation apparatus 112 will be described with reference to a sectional view of Figure 13. A tray 103 is transported while being carried on a metal member 112b of a chain 112a disposed on the opposite sides. A depression 103a is formed on the tray 103, and a pad 112c for entering the depression 103a is disposed between the chain 112a and 112a together with a gear portion 112e. The pad 112c is supported for rotation on a lift member 112e. The lift member 112e is slidably moved along a pair of slide bars 112f and 112f, and the pad member 112c is inserted into the depression 103a of the tray 103 by a pneumatic cylinder 112g as indicated by an arrow mark 192 to raise the entire tray 103. Further, a pinion 112h is held in meshing engagement with the gear portion 112e, and the pad 112c is rotated by a motor 112g to rotate the tray 103 as indicated by an arrow mark 193.

Subsequently, operation of the sampling apparatus described above will be described with reference to Figures 11 and 12. Referring to Figure 11A, a package P is stopped at a predetermined position of the surface layer yarn releasing station S101 by the stopper 106a. Then, the rotation apparatus 112 lifts the package P as indicated by an arrow mark 195 and further rotates the package P as indicated by an arrow mark 196. Subsequently, the suction mouth 113 is pivoted toward the package P as indicated by an alternate long and two short dashes line in Figure 11B. The sensor 113d detects the package P and the pivotal motion of the mouth 113 is stopped so that the desired distance ε is assured. And, the suction mouth 113 sucks an end of the yarn. After the yarn end is sucked, since the package P is being rotated, the yarn end is traversed vertically while being released. Then, the yarn end is arrested at the depression 113b of Figure 11A, and the thus arrested yarn is detected by the sensor 113c and the suction mouth 113 is retracted to the full line position of Figure 11B. And, a yarn portion 182 extending from the package P to the opening 113a is formed.

And, the first yarn catching lever 114 of Figure 12A is moved as indicated by the arrow mark 184 → the arrow mark 185 → the arrow mark 186 and moves up catching the yarn portion 182. The first yarn catching lever 114 is inclined so as to fall down as indicated by the arrow mark 187 → the arrow mark 188 so that the thus caught yarn portion 182 is transferred to the fixed second yarn catching lever 115. While the yarn portion 182 slips down along the inclined face 115b of the second yarn catching lever 115, the cutter 117 operates to cut the yarn portion 182, and an end of the yarn extending to the package is sucked into the suction pipe 116. If the suction is performed, for example, for 2.5 seconds, then the yarn of approximately 25 m is released at a high speed so that a regular yarn appears. In particular, since the yarn lifted from the package P is sucked, releasing of the yarn is performed at a high speed, and the surface layer yarn is removed in a short time. The surface layer yarn is sometimes in a damaged condition due to yarn end processing upon doffing or is not in an oiled condition, and accordingly, it is removed as a waste yarn. It is to be noted that the first yarn catching lever 114 is moved down as indicated by an arrow mark 189 and returned at the home position indicated by a solid line.

And, after the yarn from the package P becomes a regular yarn, the yarn putting aside lever 118 is pivoted by 180 degrees to put aside the yarn to the center line of the yarn splicing apparatus 119. Simultaneously, the package P moves to the sampling station S102 of Figure 11A. And, the upper yarn remaining on the yarn splicing apparatus 119 and the lower yarn from the package P are spliced to each other, and the yarn of a desired length is drawn into a test knitting machine or the like by way of the fixed guide 120. And, the package P from which sampling has been completed is fed to the yarn tying station S103, at which the yarn end cut by the yarn splicing apparatus 119 is carried to the yarn tying apparatus 130 by the suction mouth 121 which is pivoted as indicated by an arrow mark 197, and a desired tie knot 131 is formed by rotation and back and forth movement of the yarn tying apparatus 130 as indicated by an arrow mark 198.

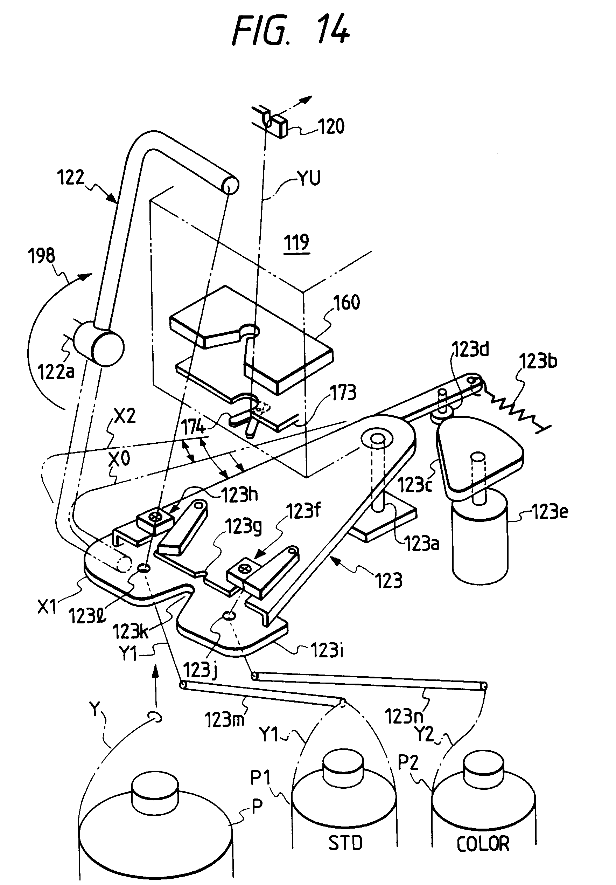

At this sampling station S102, not only successive yarn splicing of packages P fed by the transport line 101 is performed, but changing over to a package P2 on which a standard yarn for comparison is wound or another package P3 on which a color yarn for distinction is wound can be performed. To this end, a relay pipe 122 and a shifter 123 are provided for the yarn splicing apparatus 119. The changing over structure will be described with reference to a perspective view of Figure 14.

Referring to Figure 14, the shifter 123 is supported for pivotal motion by means of a shaft 123a and stops at a position defined by a tension spring 123b and a roller follower 123d which abuts with a shift cam 123c. When the shift cam 123c is rotated by a motor 123d, the shifter 123 can assume an X1 position indicated by a solid line and an X0 position and an X2 position each indicated by an alternate long and two short dashes line. A first yarn clamp 123f, a sampling yarn guide 123g and a second yarn clamp 123h are provided at an end of the shifter 123. Further, a first hole 123j, a yarn guide 123k and a second hole 1231 are provided on an end plate 123i of the shifter 123. The structure is such that a standard yarn Y1 from a package P1 passes the second hole 1231 past a pipe 123m and is clamped by the second clamp 123h (in the example shown, the second clamp 123h is opened, and then the standard yarn Y1 is sucked into and carried by the relay pipe 122 which will be described subsequently). The structure is such that a color yarn Y2 from a package P2 passes the first hole 123j past a pipe 123n and is clamped by the first clamp 123f (in the example shown, the first clamp 123f is closed and clamps the yarn Y2).

The relay pipe 122 is pivotal as indicated by an arrow mark 198 around a center shaft 122a, and when the relay pipe 122 is pivoted in a downward direction, it sucks and clamps a yarn clamped by one of the clamps 123f and 123h, and then when it rotates to its solid line position, it draws out the yarn and introduces the yarn into the yarn splicing apparatus 119. It is to be noted that the yarn splicing apparatus 119 has a fixed guide 173 on the lower side of the yarn splicing member 160, and a clamp cutter 174 is mounted on the fixed guide 173. The clamp cutter 174 has a function of cutting a yarn Y sampled from a package P and holding the yarn Y as an upper yarn YU.

Subsequently, a yarn changing over operation by the shifter 123 and the relay pipe 122 will be described. In a condition wherein a yarn Y is sampled from a package P, the shifter 123 is at the X0 position, and the yarn Y is introduced into a test knitting machine or the like by way of the fixed guide 120 under the guidance of the yarn guides 123k and 123g. After sampling of the yarn Y is completed, the clamp cutter 174 operates to cut the yarn Y from the package P and holds it as an upper yarn YU as shown in Figure 14. Then, in order to change over to the standard yarn Y1, the shifter 123 is shifted to its solid line position. Then, the standard yarn Y1 clamped by the second clamp 123h is released, and simultaneously the standard yarn Y1 is sucked into and held by the relay pipe 122. And, the relay pipe 122 is pivoted as indicated by the arrow mark 198 so that the standard yarn Y1 is introduced into the yarn splicing apparatus 119, in which it is spliced to the upper yarn YU. Then, the standard yarn Y1 of a desired length is fed out. Then, the clamp cutter 173 of the yarn splicing apparatus 119 operates, and the upper yarn YU of the standard yarn Y1 is held while at the same time the first clamp 123h is closed to return to the condition wherein it clamps the standard yarn Y1. Subsequently, in order to change over to a second color yarn Y2 of another package P2, the shifter 123 is shifted to the X2 position, and the yarn Y2 clamped by the second clamp 123 comes to the front of the relay pipe 122, whereafter the color yarn Y2 is spliced to the upper yarn YU by way of the operations described above and the color yarn Y2 of a predetermined length is fed out. Simultaneously, when the feeding out is completed, the upper yarn YU remains, and the second clamp 123f returns to the condition wherein it clamps the color yarn Y2. If a new package P is transported, then a sampling yarn Y is introduced finally into the yarn splicing apparatus 119 by the yarn putting aside lever 118 by way of the procedure described with reference to Figures 1 and 2.

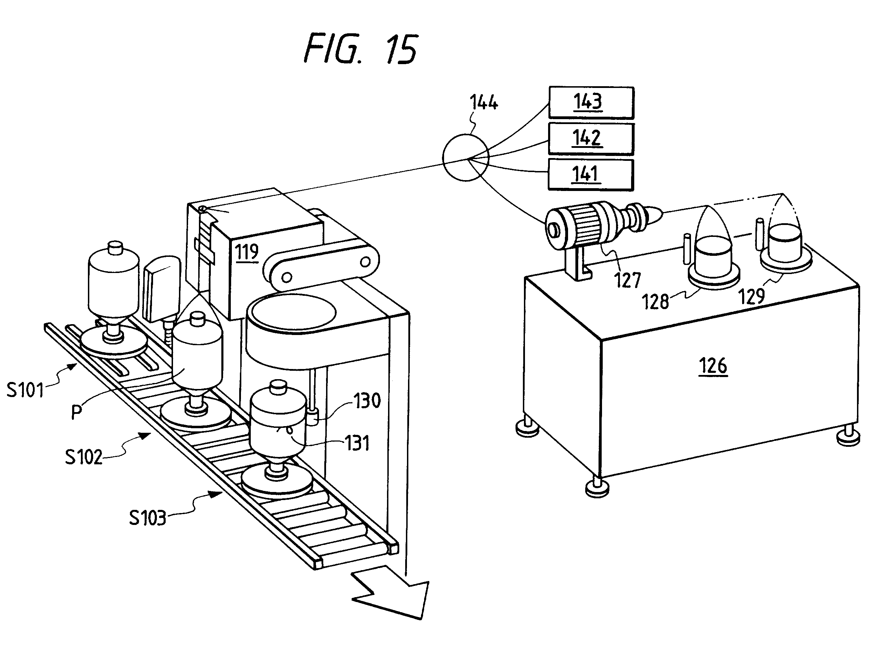

The developed view of a fabric knitted by test knitting by way of the procedure described above is such as shown in Figure 8. Further, since the yarn splicing apparatus 119 is similar to the yarn splicing apparatus 15 of the first embodiment, detailed description thereof is omitted herein. Furthermore, also the synthetic yarn transport line in which the sampling apparatus described above is incorporated is substantially similar to that of the first embodiment. The synthetic yarn transport line is different in that a sampled yarn can be introduced into another inspection apparatus, that is, a tensile test machine 141, a crimp degree measurement instrument 142 or a single yarn dyeing apparatus 143 by a changing over apparatus 144. It is to be noted that, in Figure 15, a surface layer yarn tying station S103 is constructed such that an end of a yarn of a package at the station S103 is carried from the yarn splicing apparatus 119 by a suction pipe not shown and a tie knot 131 is produced on an outer periphery of the package by the yarn tying apparatus 130 which circulates around the package P and moves back and forth.

In Figure 15, a new package P is supplied to the releasing station S101, and at the sampling station S102, the package P is drawn out toward the test knitting machine 126. At the yarn tying station S103, yarn splicing of the package P from which sampling has been completed is performed. In this manner, sampling is performed at successive steps while the package P is shifted one by one.

Since a package transport system of the present invention is constructed such that a sampling station for sampling a predetermined length of a yarn from each package is provided at a transport line for inspection, packing and so forth and a continuous yarn from the sampling station is temporarily stored into a feeder or the like so that it is used for successive inspections in accordance with the necessity as described above, a test knitting inspection and so forth are performed without by way of manual operation, and saving of labor is achieved. Further, since it can be performed readily to apply an ID for identification to a peg tray or the like of the transport line, a corresponding relationship between a fabric knitted by sampling and test knitting and packages can be established readily, and also detection of an unacceptable package can be performed automatically.

Further, since a package transport system of the present invention is constructed as a system wherein a sampling station is provided for a transport line for inspection, packing and so forth and a test knitting machine is connected to the sampling station while a standard package and a color package are installed at the sampling station so that they can be changed over such that sampling of a yarn of a predetermined amount is performed automatically and then test knitting by the test knitting machine is performed automatically and besides a standard yarn or/and a color can be continuously knitted into the test knit, a test knit which includes the standard yarn or the color yarn is performed without by way of manual operation, and saving of labor is achieved.

Besides, since a package transport system of the present invention is constructed such that a surface layer yarn releasing station for releasing and removing a yarn of a surface layer of a package is provided forwardly of a sampling station so that a yarn other than a regular yarn which has been damaged upon doffing by an automatic doffer or to which oil is not applied is automatically removed as described above, it becomes possible to construct a system wherein test knitting by a test knitting machine or the like is automatically performed subsequently and an inspection based on sampling of a regular yarn is performed without by way of manual operation, and saving of labor is achieved.

Further, according to a yarn splicing method and a yarn splicing apparatus for a yarn of the present invention, the arrangement of a pair of cutters on the opposite ends of a yarn splicing member is modified, and a suction nozzle is provided for a longer tail and a yarn is held in a taut condition during yarn splicing. Further, a tail of a spliced yarn portion, or preferably a tail on the downstream in a yarn feeding direction, is arranged to be longer than another tail on the upstream, and when the longer tail is knitted in together or the like, the spliced yarn portion appears as a linear splice and the spliced yarn portion can be discriminated at a glance by eye measurement. Consequently, distinction for each package on a fabric can be performed readily, and identification of an unacceptable package can be performed rapidly and with certainty.

Furthermore, with a sampling apparatus for a yarn of the present invention, since an end of a yarn of a surface layer of a package is sucked and lifted in a longitudinal direction of the package and high speed releasing wherein the surface layer yarn is sucked in a short time by second suction means is performed to obtain a regular yarn and then the regular yarn is introduced into a yarn splicing apparatus and is used for successive inspections, automatic inspections can be performed for all packages. Further, since a portion of a surface layer yarn at which it may possibly be damaged or a portion of a surface layer yarn which may not possibly be oiled is automatically removed and a regular yarn is sampled, results of the various inspections are reliable.

## Claims

1. A yarn package transport system, including a sampling station (S2; S102) provided for a transport line (1; 101)along which yarn packages (P) are successively transported, for sampling a yarn of a predetermined length from each package (P),
**characterized by**
a surface layer yarn releasing station (S1; S101) for releasing and removing a surface layer yarn of the packages (P) which is provided forwardly of that sampling station (S2; S102).

2. A yarn package transport system according to claim 1, wherein a yarn splicing apparatus (15; 119) for successively splicing said sampled yarns is provided in said sampling station (S2; S102).

3. A yarn package transport system acc. to claim 2, wherein a test knitting machine (16; 126) for knitting yarns spliced by said splicing apparatus (15; 119) is connected to said sampling station (S2; S102).

4. A yarn package transport system acc. to claim 2, wherein the splicing apparatus (15; 119) includes means for arranging a tail of spliced yarns to be long enough so that the spliced joint can be easily and visually found after splicing operation.

5. A yarn package transport system acc. to claim 4, wherein the yarn tail produced at one end of the splice joint is arranged to be longer than the yarn tail at the opposite end of the splice joint.

6. A yarn package transport system acc. to claim 5, wherein the tail on the downstream side with respect to the advancing direction of the yarn in which the yarn is fed for testing after yarn splicing is arranged to be longer than another tail on the upstream side.

7. A yarn package transport system acc. to claim 3, wherein a standard package (SP) on which a standard yarn for comparison is wound and/or a color package (CP) on which a color yarn for distinction is wound are provided at said sampling station (S2) such that a standard yarn and/or a color yarn can be changed over between the standard yarn or a color yarn and said sampled yarn.

8. A yarn package transport system acc. to claim 1, wherein a test machine for evaluating each yarn sampled from each package (P) is connectd in said sampling station (S2).

9. A yarn package transport system acc. to claim 1, wherein the surface layer yarn releasing station (S1, S102) includes first suction means (suction mouth 13; suction mouth 113) for sucking in an end of a yarn of a surface layer of a package (P), means for lifting the thus sucked yarn toward a longitudinal direction of the package (P), second suction means (pivotal sucker 14; suction pipe 116) for sucking-in the thus lifted yarn to release the yarn of the suface layer of the package (P'), and yarn putting aside means (levers 64, 68; lever 118) for introducing the thus released yarn into a yarn splicing apparatus (15, 119) located at the sampling station.

## Patentansprüche

1. Fadenauflaufspulen-Transportsystem mit einer Probenentnahmestation (S2; S102), die an einer Förderstraße (1; 101) vorgesehen ist, längs der Fadenauflaufspulen (P) aufeinanderfolgend transportiert werden, um einen Faden vorbestimmter Länge von jeder Auflaufspule (P) abzunehmen,
**gekennzeichnet durch**
eine Oberlächenschichtfaden-Abnahmestation (S1; S101) zum Abnehmen und Entfernen eines Oberflächenschichtfadens der Auflaufspulen (P), die vor der Probenentnahmestation (S2; S102) angeordnet ist.

2. Fadenauflaufspulen-Transportsystem nach Anspruch 1, bei dem eine Fadenverbindungsvorrichtung (15; 119) zum aufeinanderfolgenden Verbinden der entnommenen Fäden in der Probenentnahmestation (S2; S102) vorgesehen ist.

3. Fadenauflaufspulen-Transportsystem nach Anspruch 2, bei dem eine Teststrick-bzw. Wirkmaschine (16; 126) zum Stricken bzw. Wirken von durch die Verbindungsvorrichtung (15; 119) verbundenen Fäden an die Probenentnahmestation (S2; S102) angeschlossen ist.

4. Fadenauflaufspulen-Transportsystem nach Anspruch 2, bei dem die Verbindungsvorrichtung (15; 119) eine Einrichtung hat, um einen Endabschnitt der verbundenen Fäden ausreichend lange auszubilden, so daß die Verbindungsstelle nach dem Verbinden leicht und visuell aufgefunden werden kann.

5. Fadenauflaufspulen-Transportsystem nach Anspruch 4, bei dem der Fadenendabschnitt, der am einen Ende der Verbindungsstelle erzeugt wird, länger als der Fadenendabschnitt am gegenüberliegenden Ende der Verbindungsstelle ausgebildet wird.

6. Fadenauflaufspulen-Transportsystem nach Anspruch 5, bei dem der Endabschnitt auf der stromabwärtigen Seite bezüglich der Vorschubrichtung des Fadens, in der der Faden zum Testen nach der Fadenverbindung vorgeschoben wird, länger als der andere Endabschnitt auf der stromaufwärtigen Seite ausgebildet wird.

7. Fadenauflaufspulen-Transportsystem nach Anspruch 3, bei der eine Standardauflaufspule (SP), auf die ein Standardfaden zum Vergleich gewickelt ist, und/oder eine Farbauflaufspule (CP), auf die ein Farbfaden zur Unterscheidung gewickelt ist, an der Probenentnahmestation (S2) vorgesehen sind, so daß ein Standardfaden und/oder ein Farbfaden zwischen dem Standardfaden oder einem Farbfaden und dem entnommenen Faden eingewechselt werden kann bzw. können.

8. Fadenlaufspulen-Transportsystem nach Anspruch 1, bei dem eine Testmaschine zur Auswertung jedes Fadens, der jeder Packung (P) entnommen wird, mit der Probenentnahmestation (S2) verbunden ist.

9. Fadenlaufspulen-Transportsystem nach Anspruch 1, bei dem die Oberflächenschichtfaden-Abnahmestation (S1, S102) eine erste Saugeinrichtung (Saugöffnung 13; Saugöffnung 113) zum Ansaugen eines Endes eines Fadens einer Oberflächenschicht einer Auflaufspule (P), eine Einrichtung zum Heben des so angesaugten Fadens in einer Längsrichtung der Auflaufspule (P), eine zweite Saugeinrichtung (Schwenksauger 14; Saugrohr 116) zu Ansaugen des so angehobenen Fadens, um den Faden von der Oberflächenschicht der Auflaufspule (P) abzunehmen, und eine Einrichtung (Hebel 64, 68; Hebel 118) zum Beiseitelegen und Einführen des so abgenommenen Fadens in eine Fadenverbindungsvorrichtung (15, 119), die sich an der Probenentnahmestation befindet, aufweist.

## Revendications

1. Système de transport de pelotes, incluant un poste d'échantillonnage (S2 ; S102) prévu pour une ligne de transport (1 ; 101) le long de laquelle des pelotes (P) sont transportées successivement, pour un échantillonnage d'un fil d'une longueur prédéterminée de chaque pelote (P), caractérisé par un poste de relâchement de fil de la couche superficielle (S1 ; S101) pour relâcher et retirer un fil de la couche superficielle des pelotes (P) qui est réalisé en amont dudit poste d'échantillonnage (S2 ; S102).

2. Sytème de transport de pelotes selon la revendication 1, où un appareil de renforcement de fil (15 ; 119) pour renforcer successivement lesdits fils d'échantillonnage est prévu dans ledit poste d'échantillonnage (S2 ; S102).

3. Système de transport de pelotes selon la revendication 2, où un métier à tricoter pour essais (16 ; 126) pour tricoter des fils renforcés par ledit appareil de renforcement (15 ; 119) est relié audit poste d'échantillonnage (S2 ; S102).

4. Système de transport de pelotes selon la revendication 2, où l'appareil de renforcement (15 ; 119) comporte un moyen pour agencer un bout de fil renforcé pour qu'il soit suffisamment long pour que le point de jonction renforcé puisse être trouvé facilement et visuellement après l'opération de renforcement.

5. Système de transport de pelotes selon la revendication 4, où le bout de fil produit à une extrémité du point de jonction renforcé est agencé pour être plus long que le bout de fil à l'extrémité opposée du point de jonction renforcé.

6. Système de transport de pelotes selon la revendication 5, où le bout sur le côté aval par rapport à la direction d'avance du fil dans laquelle le fil est amené en vue d'un essai après le renforcement du fil est agencé pour être plus long qu'un autre bout sur le côté amont.

7. Système de transport de pelotes selon la revendication 3, où une pelote standard (SP) sur laquelle un fil standard pour une comparaison est enroulé et/ou une pelote en couleur (CP) sur laquelle un fil de couleur en vue d'une distinction est enroulé, sont prévus audit poste d'échantillonnage (S2) de telle sorte qu'un fil standard et/ou un fil en couleur peut être changé entre le fil standard ou un fil de couleur et ledit fil échantillonné.

8. Système de transport de pelotes selon la revendication 1, où une machine pour essais pour évaluer chaque fil échantillonné de chaque pelote (P) est connectée dans ledit poste d'échantillonnage (S2).

9. Système de transport de pelotes selon la revendication 1, où le poste de relâchement de fil de la couche superficielle (S1, S102) comporte un premier moyen d'aspiration (embouchure d'aspiration 13 ; embouchure d'aspiration 113) pour aspirer une extrémité d'un fil d'une couche superficielle d'une pelote (P), un moyen pour relever le fil ainsi aspiré vers une direction longitudinale de la pelote (P), un deuxième moyen d'aspiration (organe d'aspiration pivotant 14 ; tuyau d'aspiration 116) pour aspirer le fil ainsi relevé afin de relâcher le fil de la couche superficielle de la pelote (P'), et un moyen d'écartement de fil (leviers 64, 68 ; levier 118) pour introduire le fil ainsi relâché dans un appareil de renforcement de fil (15, 119) situé au poste d'échantillonnage.
